(19) Europäisches Patentamt
European Patent Office
Office européen des brevets

(11) Publication number: 0 399 127 A1

(12) # EUROPEAN PATENT APPLICATION

(21) Application number: 89810383.3

(22) Date of filing: 23.05.89

(51) Int. Cl.5: G01N 33/58, G01N 33/542, G01N 33/94

(43) Date of publication of application: 28.11.90 Bulletin 90/48

(84) Designated Contracting States: AT BE CH DE ES FR GB GR IT LI LU NL SE

(71) Applicant: Pharmacia ENI Diagnostics Inc.
350 Passaic Avenue
Fairfield New Jersey 07007-2803(US)

(72) Inventor: Siddiqi, Iqbal
7, Chemin des Vignettes
CH-1231 Villette(CH)
Inventor: Brochot, Jean
L'Agnellu Feigères
F-74160 St-Julien-en-Genevois(FR)

(74) Representative: Dousse, Blasco et al
7, route de Drize
CH-1227 Carouge/Genève(CH)

(54) **Homogeneous immunochemical method for determining haptens by means of ion selective electrodes.**

(57) This test involves reacting the hapten with an antibody in the presence of a dehydrogenase labelled hapten.

The free label is used as signal generator in a reaction with an enzyme substrate in the presence of Nicotinamide Adenine Dinucleotide (NAD) which is converted to NADH. NADH is determined electrometrically by its action on the peroxidase catalyzed splitting of fluoride ions from aromatic fluoro compounds using a fluoride selective electrode. No separation step is necessary and the test is homogeneous.

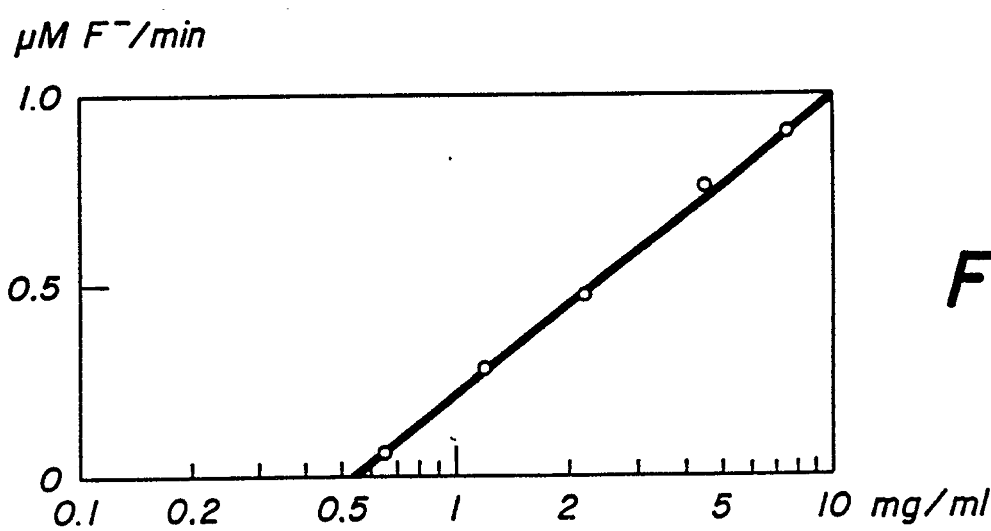


FIG. 1

# HOMOGENEOUS IMMUNOCHEMICAL METHOD FOR DETERMINING HAPTENS BY MEANS OF ION SELECTIVE ELECTRODES

The present invention concerns homogeneous immunoassays, more particularly enzyme multiplied immunoassay techniques (EMIT®), and enzyme linked immunosorbent assays (ELISA).

The terms homogeneous immunoassay may be defined as an immunoassay in which the extent to the antigen-antibody reaction can be determined without physical separation of the free and bound forms. This term is usually used for immunoassays such as enzyme and fluorescence immunoassays in which labeled substances (markers) are used. Homogeneous immunoassays are widely used as routine tests because the procedures involved are simple. The principle of homogeneous immunoassay is based on changes in signals of the indicators by the antigen-antibody reaction.

Homogeneous nonisotopic immunoassays are classified into several groups according to the mechanisms used for detection of the antigen-antibody reaction. In the homogeneous immunoassay based on marker-labeled antigen, a change in activity of a marker coupled with antigen, caused by binding to an antibody, is measured. When unlabeled antigen is added, it competes with marker-labeled antigen for binding to the antibody. Usually the free form of marker-labeled antigen has increased activity. Any change in the activity of the markers can be stoichiometrically related to the concentration of the unlabeled antigen in the sample. The procedure in this method is simple, but the method is usually not applicable to antigens that are macromolecules. Immunoassays included in this category are as follows: homogeneous enzyme immunoassay (an early example was developed by Rubenstein et al. in 1972 for morphine), enzyme membrane immunoassay, fluorescence polarization immunoassay, fluorescence quenching immunoassay, fluorescence enhancement immunoassay, fluorescence protection immunoassay, fluorescence immunoassay based on excitation by internal reflection spectroscopy, spin immunoassay, viroimmunoassay, and particle immunoassay. Alternatively, marker-labeled antibody can be used to measure antibody.

Homogeneous enzyme immunoassay introduced by Rubenstein is one immunoassay technique (commonly known as the EMIT® system) which depends upon a change in the specific enzyme activity when antibody is bound to enzyme labeled antigen. The activity of the unseparated assay mixture responds to the proportion of enzyme labeled antigen to which antibody is bound. Thus, no separation step is required. The consequent saving in the time required to do each measurement and the nature of the required spectrophotometric equipment translates into a considerable reduction in the cost of performing the test.

Since the original homogeneous enzyme immunoassay was described, other related homogeneous methods have appeared. Recent applications of the EMIT® method concern haptens and proteins.

Three distinct variants of the EMIT® principle have been devised and applied commercially. The early EMIT® assays employed lysozyme as the enzyme label. The natural substrate for this enzyme is the peptidoglycan of the cell wall of certain bacteria. When lysozyme acts on a suspension of Micrococcus luteus, the turbidity of the suspension is reduced thus providing a convenient measure of enzyme activity. Lysozyme-haptens conjugates are active with the bacterial suspension. When hapten antibodies bind to an enzyme-hapten conjugate, however, a steric barrier is presented to the bulky substrate. Antibody binding thus inhibits enzyme activity by up to 98%. This effect is made use of in the EMIT® assays for drugs of abuse.

Enzymes with small substrates have also been successfully employed in EMIT® assays. Hapten conjugates of glucose-6-phosphate dehydrogenase and malate dehydrogenase are inhibited up to 80% by excess hapten antibodies. The mechanism of inhibition in the case of malate dehydrogenase was investigated and it was concluded that inhibition was due to conformational effects. Most likely, an inhibitory conformational effect is induced by antibody binding to haptens linked to certain "active" groups on the enzyme surface. Alternatively, a con formational change required for activity is prevented by antibody binding. The dehydrogenase enzymes are also used in EMIT® assays for the measurement of compounds of medical importance such as opiates, barbiturates, amphetamines, methadone, cocaine metabolites, benzodiazepine, cannabinoid drugs, phenylcyclidine, antidepressants, thyroid drugs, antimicrobial drugs, antiepileptic drugs, cardioactive drugs and many other therapeutic drugs.

The present invention deals more particularly with the application of ions selective electrodes (ISE) as measuring means in EMIT® immunoassays using dehydrogenase enzymes as markers. Such technology is currently used for determining haptens, such as these listed above, in biological fluids, for instance blood sera or urine.

As explained above, EMIT® is a competitive type homogeneous immunoassay technique in which the analytical sample containing the hapten to be ascertained is immuno-reacted with a known quantity of anti-hapten in the presence of a given quantity of labeled hapten. In EMIT® technology, the enzyme is the signal generator, said signal being provided by the reaction with a suitable enzyme substrate added to the reaction medium. In these tests, the generation of signal by the antibody bound enzyme is inhibited and therefore no separation between immuno-bound and free signal generator is necessary, whereby the test is homogeneous.

In the determination of many haptens, including digoxin and theophyllin, convenient enzyme labels are the co-enzyme dependent dehydrogenase enzymes, said co-enzymes being nicotinamide adenine dinucleotide (NAD). (For details, see Methods in Biology and Medicine, Vol. 4, Immunoassays, by R.M. NAKAMURA et al., Ed. Alan R. Liss, Inc. New York (1980). For instance in a digoxin test kit commercialized by SYVA DIAGNOSTICS LTD,(Great Brittain), the sample is immuno-reacted with anti-digoxin in the presence of digoxin labelled with glucose-6-phosphate dehydrogenase (G6PDH). The free label is thereafter determined by its action in the oxydation of glucose-6-phosphate in the presence of NAD which is quantitatively converted to NADH, the characteristic absorption changes related to NAD→ NADH being measured spectrophotometrically.

Although this test has merits, the absorption measurements are often hampered by the presence, in the sample of extraneous coloured compounds which variably interfere with the measurements.

Therefore efforts have been made to supplement or replace absorption measurements by means insensitive to the presence of undesirable side products.

Such means, have now been found and have become the key feature of the test method of the present invention as outlined in annexed claim 1.

Basically, the improvement here consists in using an ion selective electrode as probing means, more especially a fluoride sensitive electrode, and generating fluoride ions in proportion to the amount of NADH formed in the reaction, said fluoride ions being then measured by means of the ISE.

The reaction schemes are given below in which Ha is the hapten antigen to be measured, Ha* is the hapten labeled with the dehydrogenase signal generator, G6P is glucose-6-phosphate, Ab is the hapten-antibody, PER is horse-radish peroxidase, and FP is the aromatic fluorocompound.

(1) Ha (Ha*) + Ab(lack) → HaAb (Ha*Ab) + free Ha*

$$(2)\ G6P + NAD \xrightarrow{Ha*} \text{Gluconolactone-6-phosphate} + NADH$$

$$(3)\ NADH + FP \xrightarrow{PER} F^- + NAD$$

The liberated fluoride ions $F^-$ can then be quantitatively measured electrometrically with a fluoride-ion selective electrode.

In step (3), $Mn^{+2}$ and 4AAP are preferably added to increase the sensitivity of the determination. The concentration of $Mn^{+2}$ ions is preferably between $4 \times 10^{-4}$ and $10^{-2}$M and the concentration of 4AAP is preferably between $10^{-5}$ to $10^{-3}$M. The reaction may be carried out in buffered medium at pH 5 to 8.5. Horse radish peroxidase is the preferred peroxidase enzyme.

Preferably, the reactions are effected in cacodylate buffer (0.1 - 0.5 M) at pH 7; however other buffers may also be used, although less conveniently, e.g. phosphate, acetate, borate, tris buffers; pH 7 is preferred, although values below or above neutrality (e.g. 5 - 8.5) are possible but less convenient.

Usually, but not necessarily so, the test involves diluting the sample of serum in buffer, adding thereto in quick succession the various reagents including the enzyme label, the fluorocompound, the enzyme substrate and the other ingredients in separate or premixed form, plus the fluoride selective electrode, then allowing the reactions to proceed under agitation which recording the electrode potential. The rate of change of the potential (during the significant reaction period) is proportional to the amount of fluoride ions generated.

Basically, an excess of an aromatic fluoro compound and peroxidase are incorporated to a buffer medium containing the reduced co-enzyme to be determined and the other ingredients participating to the various reactions, and then the medium is stirred in the presence of air. The result is a breakage of the F-C bond in the fluoro compound and corresponding release of $F^-$ ions at a rate proportional to the quantity of co-enzyme present. Since in the presence of the dehydrogenase enzyme NADH continues to be produced (although at a slower rate when the initial measurement is over), making "end-point" type measurements is

not possible. "Fixed-time" analysis is however possible by effecting the determination after a "fixed" given time, which is always the same for a set of analyses of the same type. In general, however, it is preferable to measure the release rates of $F^-$ ions in the kinetic mode under well-standardized conditions in order to obtain good reproducibility of measurements. For example, the measurement of the gradients of the rate curves (which of course depends on certain reaction parameters as well as on the concentration of substance to be measured) will advantageously be made after a certain latency time (incubation), the time being kept the same during a set of comparative tests. However, the latency time may be slightly different from one analysis to the other since the maximum rate is obtained more quickly when the NADH concentration is higher. The buffers can be the usual buffers at pH 5 to 7.5, inter alia acetate, "tris", cacodylate, etc. The latter is preferred since it can rapidly stabilize the response of the fluoride electrode. It has also been found that where cacodylate buffer is employed, the reaction rate (the breakage of the C-F bond) depends on the dimethylarsenate concentration in the buffer. Preferably the cacodylate buffer has a molar concentration of 0.1 to 0.5 M.

The concentration of release $F^-$ ions is preferably determined by using an electrode sensitive to $F^-$ ions but inert towards to other kinds of ions. The electrode may advantageously be an electrode selective to $F^-$ ions and of type 96-09 produced by ORION RESEARCH INC, Cambridge, Mass., USA. Other electrodes may be equally suitable, however. All details regarding use of these electrodes for determining $F^-$ ions may be found in document EP-A 20 623 incorporated here by reference.

Briefly, a standard curve is preferably referred to in order to determine the release rate of $F^-$ ions after reaction of an unknown sample. A standard curve can be obtained as described hereinbefore by determining a set of samples containing known concentrations of hapten. The release of $F^-$ ions is recorded for each sample and the gradient of the kinetic curves is measured at a time (the same of course for each sample) when the rate curves are almost straight lines. The gradients are shown on a graph in relation to the concentrations of co-enzymes, so as to obtain a standard reference curve. The measured electrometric parameters used for preparing the kinetic curves can be the voltage readings of the electrometric system used together with the fluorine electrode (mV), or preferably the corresponding values of $[F^-]$ which can be calculated by the Nernst equation, which is the present case has the following form:

$E = E' - S.\log [F^-]$

where E is the measured voltage and $E'$ is an experimentally determined constant belonging to the system and including the activity factors and potentials of liquid junctions. S is the "Nernst slope" which is a constant equal to about 57.5 mV (in the cacodylate buffer at pH 7.5) for a variation of 10 units in the $F^-$ concentration, the latter being expressed in mols/l. If the $[F^-]$ values calculated from the above relation are used in the kinetics instead of the values in mV, the resulting curves are closer to straight lines and their gradient is easier to establish, so that more accurate reference graphs can be drawn.

The following are examples of aromatic compounds fluorinated on the ring and suitable for the present invention: 2-, 3- and 4-fluorophenols, tetrafluorophenol, pentafluorophenol and p-fluoroaniline; 4-fluorophenol and penta-fluorophenol are preferred.

The examples below illustrate the invention with reference to the annexed drawing.

Fig 1 is a calibration graph showing the relation between the concentration of digoxin in standards of serum and the corresponding rate of fluoride formation due to the presence of NADH which results from the oxidation of G6P and NAD catalyzed by the G6PDH unbound label.

Fig 2 is a graph showing the absorbance at 340 nm of standards of digoxin analyzed by the SYVA EMIT® method.

Fig 3 is a graph similar to that of Fig 1, but relating to measuring theophyllin.

## Example 1

Analysis of Digoxin.

In this example as in others, reagents were prepared starting with ingredients from the EMIT® test kit for analyzing digoxin marketed by SYVA DIAGNOSTICS, Great Britain.

The SYVA kit contains reagents A and B and a pretreatment solution which serves for preconditioning the serum sample before analysis. The utility of this preconditioning step in connection with the present invention has not been assessed.

Reagent A contains the following reagents: an antibody against digoxin raised in sheep, glucose-6-phosphate and NAD, all three reagents are in concentration per ml sufficient to effect the titrations.

Reagent B contains labelled digoxin, i.e. digoxin marked with glucose-6-phosphate dehydrogenase (G6PDH) in quantities corresponding to A.

The SYVA kit also contains digoxin calibration sera of which aliquots were taken to make standards with digoxin content from zero to 10 ng/ml.

The buffer used was 0.1 M cacodylate at pH 7.0 (adjusted with 1N NaOH).

The following solutions were prepared

A. Reagent A of SYVA (0.2 ml) was diluted with 4.8 ml of buffer,

B. Reagent B of SYVA (0.2 ml) was diluted with 1.8 ml of buffer,

C. 125 U/ml of HRP in buffer,

D. The following solutions were mixed together: 0.5 ml of 1M $MnCl_2$ in water; 0.5 ml of $5.10^{-2}$M 4AAP in water; 2 ml of 0.25 M 4-fluorophenol (4FP) in water; buffer 2 ml. This mixture formed solution D (total 5 ml; $10^{-1}$M $MnCl_2$).

To 0.1 ml of standard digoxin calibration serum was added 25 µl of preconditioning solution from SYVA, after and 5 min incubation at room-temperature, 0.65 ml of solution A. The mixture was incubated at 35°C for 15 min and was allowed to rest at room temperature for a while.

Then 0.25 ml of solution B was added and the mixture (total 1.025 ml) again incubated at 35°C for 17 min.

0.900 ml of this solution was pipetted into one 3.5 ml well of a 24 well Limbro tissue culture plate provided with a small bar magnet and, under agitation, 100 µl of solution D were added (note that the $Mn^{+2}$ concentration there is now $10^{-2}$M) followed by immersion therein of the fluoride electrode. This electrode was connected to a Model 619 Keithly Electrometer data being acquired via an IEEE-488 interface on an Apple II+ microcomputer.

After 2 min stabilization time, 20 µl of solution C (HRP) was added and the voltage change in the cell was recorded for about 200 sec. The curves obtained were remarkably straight and the slope in direct proportion to the signal generated by the enzyme label. Since this is a competitive test, the blanks (zero digoxin) still gave a signal, either because the amount of antibody used is not sufficient to quench all label even in the total absence of test digoxin or because the bound label is not completely mute. The results are therefore reported in terms of slopes of the kinetic curves from which the value of the blank has been substracted. These results are presented graphically in fig 1. The concentrations of digoxin in ng/ml are plotted on a logarithmic scale (X-axis) against the rate of liberation of $F^-$ in µmole/L/min, normal scale (Y-axis). It can be seen that several standard mesurements form a reasonably straight line.

This chart was thereafter used for the determination, by comparison, of unknown samples of blood serum which were analyzed according to the above procedure. Satisfactory results were experienced.

As a matter of comparison with the prior art, experiments were run using the SYVA EMIT® standards and following the SYVA procedure under the same ordinary laboratory conditions used in the method of the invention. Normally the SYVA procedure requires very sophisticated operating conditions such as using a thermostatted spectrophotometer and automated pipetting apparatus which are expensive and require highly skilled operators. Fig. 2 represents graphically the results obtained and shows that this method of the prior art is more difficult to operate under ordinary unsophisticated conditions and provides less reproducible results than the method of invention.

Example 2

Determination of theophyllin.

As in the previous example, the preliminary reagents were taken from an EMIT® kit marketed by SYVA. The SYVA reaction medium, a Tris-HCl buffer was not used but replaced by a 0.1 M cacodylate buffer adusted to pH 7 with 0.1 N NaOH.

The following solutions were prepared:

E. 50 µl of the SYVA reagent A (anti-theophyllin raised in sheep + substrate glucose-6-phosphate + co-enzyme in oxidized form) was diluted with 250 µl of buffer

F. The same dilution was made with SYVA reagent B (conjugate of theopyllin and G6PDH).

G. Peroxidase solution containing 125 U/ml in buffer

H. This was the same as solution D in example 1

I. Samples of serum containing standards of digoxin (0 to 100 µg/ml) diluted 1 to 5 in buffer

The operational testing technique was similar to that of example 1 with the exception of incubation at

35° C which was not necessary.

100 µl of the diluted standard serum plus 300 µl of buffer were added to a 3.6 ml well from a "Limbro tissue culture plate", together with 300 µl of solution E, 100 µl of solution H and 20 µl of the peroxidase solution G.

The fluoride electrode was immersed in the liquid phase under agitation with a magnetic bar and 300 µl of solution F were added.

After about 1-2 min stabilization time, the potential of the electrode was recorded for about 2 min.

The results, in terms of the slopes of the kinetic curves (expressed in µmole $F^-$ L min) for several theophyllin standard concentrations are plotted to give the graph shown in fig 3.

This graph was used as a calibration chart for subsequently determining theophyllin in unknown samples. The results correlated well with known methods.

## Claims

1. A method for immuno-determining haptens in fluid analytes, by an immuno test comprising the steps of:

a) reacting said analyte with a specific antibody partner of the hapten to be measured in the presence, as a competitive reactant, of an enzyme labelled hapten as signal generator;

b) oxidizing a substrate in the presence of co-enzyme NAD (Nicotinamide Adenine Dinucleotide), with quantitative formation of NADH, said oxidizing being catalyzed by said enzyme label of the hapten;

c) reacting the NADH formed with oxygen in the presence of peroxidase, and an aromatic fluorocompound, this reaction leading to the breaking of the carbon-fluorine bond of said fluorocompound and the corresponding release of said fluorine in the form of fluoride ion in proportion to the amount of NADH;

d) potentiometrically measuring the fluoride ion with an ion selective electrode wherefrom rate data indicative of the amount of hapten to be determined are obtained;

2. The method of claim 1, in which steps a) to d) are effected concomittently in one phase medium without separation stages, whereby the immunotest is homogeneous.

3. The method of claim 2, in which the fluid analyte is of biological origin, e.g. blood serum or urine.

4. The method of claim 2, in which the hapten is selected from the group consisting of opiates, barbiturates, amphetamines, methadone, cocaine metabolites, benzodiazepine, cannabinoid drugs, phenylcyclidine, antidepressant drugs, therapeutic drugs, thyroid drugs, anti-microbial drugs, antiepileptic drugs, cardioactive drugs, digoxin, theophyllin, etc.

5. The method of claim 1, in which the enzyme label is a co-enzyme-dependent dehydrogenase, e.g. glucose-6-phosphate dehydrogenase, malate dehydrogenase and the like.

6. The method of claim 1, in which said potentiometric measurements are effected in the kinetic mode or the fixed time non-equilibrium mode.

7. The method of claim 2, in which the medium is buffered at pH 5 to 8.5.

8. The method of claim 7, in which the buffer is 0.1 -0.5 M cacodylate and the pH is 7.0.

9. The method of claim 1, in which step c) is performed in the presence, in the medium, of manganese ions and 4-amino-antipyrine.

10. The method of claim 9, in which the concentration of Mn ions is 4 x $10^{-4}$ to $10^{-2}$M and the concentration of the 4-aminoantipyrine is $10^{-5}$ to $10^{-3}$M.

11. The method of claim 1, in which the peroxidase is horse-radish peroxidase.

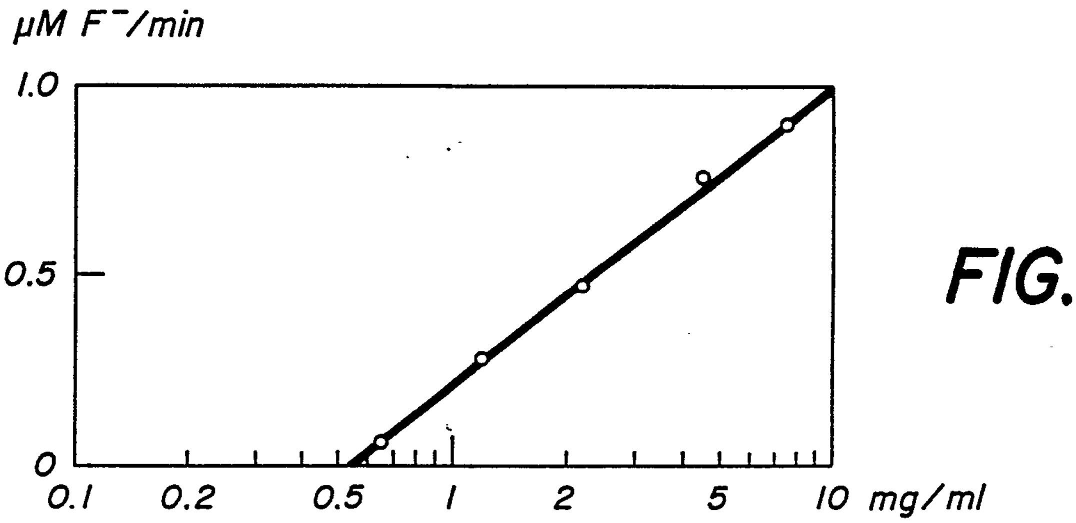


FIG. 1

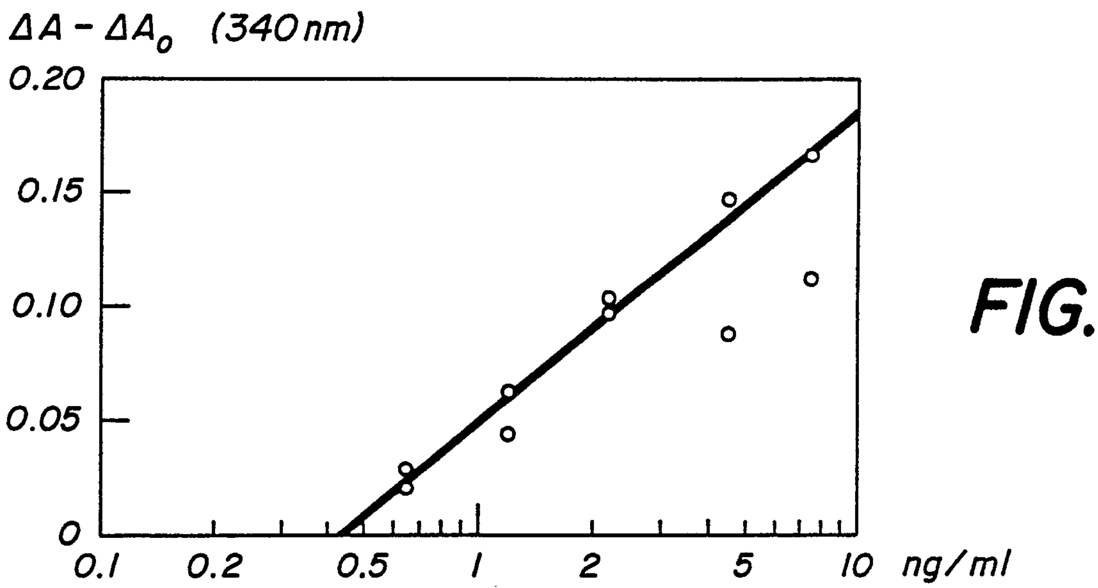


FIG. 2

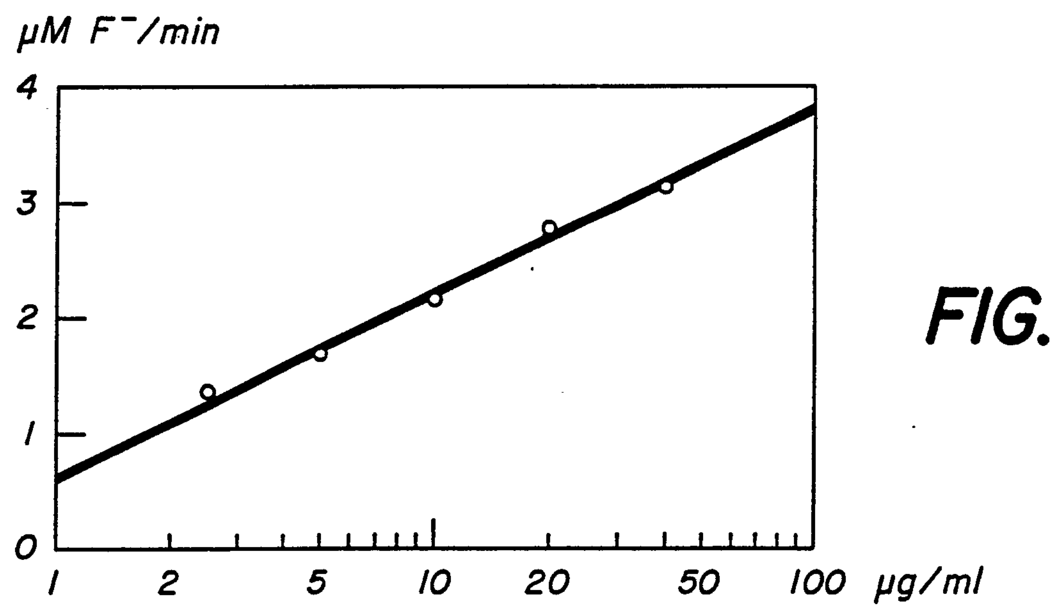


FIG. 3

European Patent Office

# EUROPEAN SEARCH REPORT

Application Number

EP 89 81 0383

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | Am. J. Clin. Pathol. vol. 81, no. 5, 1984, pages 602-610; B. HEPLER et al.: "Homogeneous enzyme immunoassay of acetaminophen in serum" * Page 602 * | 1-11 | G 01 N 33/58<br>G 01 N 33/542<br>G 01 N 33/94 |
| Y | WO-A-8 605 517 (BATTELLE MEMORIAL INSTITUTE) * Pages 1-10; pages 17-18 * | 1-11 | |
| Y | JOURNAL LIQUID CHROMATOGRAPHY, vol. 6, no. 12, 1983, pages 2141-2156; K.R. WEYMEYER et al.: "Liquid chromatography with electrochemical detection of phenol and NADH for enzyme immunoassay" * Abstract; introduction; page 2144, line 20 - page 2145, line 13; page 2148, line 16 - page 2150, line 15 * | 1-8,11 | |
| Y | EP-A-0 227 073 (F. HOFFMANN - LAROCHE & CO.) * Page 1, line 1 - page 8, line 5 * | 1-8,11 | **TECHNICAL FIELDS SEARCHED (Int. Cl.5)** |
| A | EP-A-0 125 368 (BATTELLE MEMORIAL INSTITUTE) * Page 5, line 21 - page 6, line 26 * | 1 | G 01 N<br>C 12 Q |
| A | WO-A-8 603 837 (IQ (BIO) LTD) * Abstract; page 5, line 2 - page 6, line 20; page 11, line 9 - page 12, line 24 * | 1 | |

The present search report has been drawn up for all claims

| Place of search | Date of completion of the search | Examiner |
|---|---|---|
| THE HAGUE | 23-01-1990 | HITCHEN C.E. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document